# EUROPEAN PATENT SPECIFICATION

(11) **EP 4 218 866 B1**
(45) Date of publication and mention of the grant of the patent: **29.01.2025**
(21) Application number: 23164457.6
(22) Date of filing: 19.06.2020
(51) Int. Cl.: A61M 11/02, A61M 11/04, A61M 15/00, A61M 15/06

(54) **AEROSOL DELIVERY DEVICE**
AEROSOLABGABEVORRICHTUNG
DISPOSITIF DE DISTRIBUTION D'AÉROSOL

(30) Priority: 21.06.2019 EP 19181686; 21.06.2019 EP 19181694; 21.06.2019 EP 19181683
(43) Date of publication of application: 02.08.2023
(62) Divisional of application: 20733438.4
(73) Proprietor: Imperial Tobacco Limited, Bristol, BS3 2LL (GB)
(72) Inventor: SAJTOS, Tamas, Liverpool, L24 9HP (GB); AUSTIN, Andrew, Liverpool, L24 9HP (GB); LOMAS, Pete, Liverpool, L24 9HP (GB); LORD, Chris, Liverpool, L24 9HP (GB)
(74) Representative: Mewburn Ellis LLP

(56) References cited:
- WO-A1-2011/050943
- WO-A1-2014/012907
- WO-A1-2016/050244
- WO-A1-2016/124741
- WO-A1-2017/185051
- GB-A- 2 566 766

## Description

### Technical Field

The present disclosure relates to an aerosol delivery device and system, and particularly, although not exclusively, to an aerosol delivery device which can be assembled more reliably.

### Background

One form of an aerosol delivery device is a smoking-substitute system (or device), which is an electronic system that permits the user to simulate the act of smoking by producing an aerosol or vapour that is drawn into the lungs through the mouth and then exhaled. The inhaled aerosol or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol or vapour of similar appearance to the smoke exhaled when using such traditional smoking or tobacco products.

One approach for a smoking substitute system is the so-called "vaping" approach, in which a vaporisable liquid, typically referred to (and referred to herein) as "e-liquid", is heated by a heating element to produce an aerosol/vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute system includes a mouthpiece, a power source (typically a battery), a tank for containing e-liquid, as well as a heating element. In use, electrical energy is supplied from the power source to the heating element, which heats the e-liquid to produce an aerosol (or "vapour") which is inhaled by a userthrough the mouthpiece. For example, WO2016050244A1 discloses an electronic nicotine delivery system (ENDS) comprising a mouth piece (MP), an atomizer arrangement (AA), a power supply (PS), a nicotine container (NC) and an additive container (AC). For example, WO2017185051A1 discloses a system for aerosolization of compartmentalized materials wherein the system performs operations comprising vaporizing, by a heater, at least a portion of a first material to form a first vapor. For example, WO2016124741A1 discloses an aerosol generating system, the system comprising: aerosol generating means; aerosol delivery means; and an aerosol guiding device. For example, GB2566766A discloses an aerosol delivery device comprising a porous nib.

Vaping smoking substitute systems can be configured in a variety of ways. For example, there are "closed system" vaping smoking substitute systems, which typically have a sealed tank and heating element. The tank is pre-filled with e-liquid and is not intended to be refilled by an end user. One subset of closed system vaping smoking substitute systems include a base unit which includes the power source, wherein the base unit is configured to be physically and electrically coupled to a consumable including the tank and the heating element. The consumable may also be referred to as a cartomizer. In this way, when the tank of a consumable has been emptied, the consumable is disposed of. The base unit can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the system can be used multiple times.

An example vaping smoking substitute system is the myblu^{®} e-cigarette. The myblu^{®} e-cigarette is a closed system which includes a base unit and a consumable. The base unit and consumable are physically and electrically coupled together by pushing the consumable into the base unit. The base unit includes a rechargeable battery. The consumable includes a mouthpiece, a sealed tank which contains e-liquid, as well as a heating element, which for this system is a heating filament coiled around a portion of a wick. The wick is partially immersed in the e-liquid, and conveys e-liquid from the tank to the heating filament. The device is activated when a microprocessor on board the base unit detects a user inhaling through the mouthpiece. When the system is activated, electrical energy is supplied from the power source to the heating element, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the vaping approach, e-liquid is heated by a heating element to produce an aerosol/vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices, it is desirable to be able to assembly the component parts of the device in an easy and reliable manner.

In some smoking substitute devices/systems, liquid used to form an aerosol can leak from the device and/or can collect in or on parts of the device. When such liquid collects in parts of the device that are within an airflow path of the device, such liquid can be entrained in an airflow in the airflow path. It may also be desirable to increase the degree of sanitation relating to the device.

The present disclosure has been devised in light of the above considerations.

### Summary

The invention is set out in the appended claims.

The provision of an aerosolisation chamber having a uniform cross-sectional area, as opposed to e.g. a conical chamber, may reduce or avoid the build-up of aerosol precursor at the mouthpiece aperture. For example, a conical chamber can diverge from the aerosol generating portion of the liquid transfer element, which may result in a space between the wall(s) defining the chamber and the liquid transfer element that is larger than would otherwise be the case of a non-diverging (i.e. uniform cross-section) chamber. This larger space may allow for a greater build-up of aerosol precursory, so as to result in a larger propensity for leakage from the device and may also result in a greater propensity for large droplets of aerosol precursor to be entrained in airflow from the device. Thus, the provision of a uniform cross-section chamber may result in a more consistent droplet size in the aerosol delivered by the device.

Similarly, when the space between the chamber walls and the aerosol generating portion is minimised, the surface tension of any aerosol precursor between the chamber walls and the aerosol generating portion may be more likely to be sufficient to retain the aerosol precursor in the device.

As the aerosolisation chamber extends from the mouthpiece aperture, it may otherwise be considered an outlet (e.g. a mouthpiece outlet) of the device.

The aerosol generating portion of the liquid transfer element has a larger transverse cross-sectional area (taken at a location adjacent the conveying portion) than a transverse cross-sectional area (taken at a location adjacent the aerosol generating portion) of the conveying portion. In this respect, the aerosol generating portion may define an enlarged portion of the aerosol generator. The aerosol generating portion may have a circular transverse cross-sectional shape. The conveying portion may have a circular transverse cross-sectional shape. In this respect, the aerosol generating portion may have a larger radius than the conveying portion.

Due to the aerosol generating portion being larger than the conveying portion, a transversely (e.g. radially) extending transition surface may be defined between the conveying portion and the aerosol generating portion. The transition surface may define an outward step from the conveying portion to the aerosol generating portion. The transition surface may have a concave profile. Hence, the transition surface may form a smooth continuous transition with an outer circumferential surface of the conveying portion. On the other hand, there may be a (substantially) sharp transition between the transition surface and an outer circumferential surface of the aerosol generating portion. In this respect a leading edge of the aerosol generating portion may be defined between the transition surface and the outer circumferential surface of the aerosol generating portion.

The aerosolisation chamber may have a cylindrical shape. For example, the aerosolisation chamber may have a circular cylinder shape (i.e. having a circular transverse cross-section) or an elliptical cylinder shape (having an elliptical transverse cross-section). A transverse cross-sectional shape of the aerosol generating portion may correspond to a transverse cross-sectional shape of the aerosolisation chamber (i.e. they may both be circular or elliptical). In this way, the aerosol generating portion and a wall defining the aerosolisation chamber may be concentrically arranged.

The longitudinal length of the aerosolisation chamber may be between 6 mm and 3 mm, for example between 5 mm and 4 mm, e.g. about 4.5 mm.

The aerosol generating portion may be fully received in the aerosolisation chamber. A portion of the conveying portion (i.e. a portion adjacent the aerosol generating portion) may be received in the aerosolisation chamber. Alternatively, only a portion of the aerosol generating portion (or liquid transfer element) may be received in the aerosolisation chamber (i.e. at an upstream end of the aerosolisation chamber). Thus a portion of the aerosolisation chamber (e.g. at or adjacent to the mouthpiece aperture and downstream of the aerosol generating portion) may not include the aerosol generating portion.

An airflow path is defined between a wall defining the aerosolisation chamber and the liquid transfer element. Where the aerosolisation chamber is cylindrical, the airflow path may be annular. The airflow path includes a constricted region defined by the aerosol generating portion. The transverse/radial distance between the aerosol generating portion and the wall of the aerosolisation chamber at the constricted region may be less than 0.5 mm, for example less than 0.4 mm, or e.g. less than 0.3 mm.

The constricted region may define the narrowest part of the airflow path through the aerosolisation chamber. This constriction of the airflow passage increases the velocity of air/vapour passing through the aerosolisation chamber. In this respect, the constriction may be referred to as a Venturi aperture. The constriction may have a toroidal shape (i.e. extending about the aerosol generating portion of the liquid transfer element). The toroidal shape may, however, be interrupted by supports (e.g. projections, ribs, etc.) protruding inwardly from wall(s) of the aerosolisation chamber to support the aerosol generating portion in the aerosolisation chamber.

In addition to increasing the airflow velocity, the constriction reduces the air pressure of the airflow flowing through the constriction (i.e. in the vicinity of the aerosol generating portion). This low pressure and high velocity facilitate the generation of an aerosol from the aerosol precursor held in the aerosol generating portion (i.e. conveyed from the storage chamber by the liquid transfer element). This aerosol is entrained in the airflow passing through the constriction and is discharged from the mouthpiece aperture of the aerosol delivery device.

The airflow path may have an expansion region downstream of the constricted region, in which a cross-sectional area of the airflow path (e.g. gradually) increases in the downstream direction. The expansion region may be defined by a tapered section of the aerosol generating portion. The tapered section may taper inwardly in a direction from the constricted region to a downstream (distal) end of the aerosol generating portion. The taper may have a curved profile (i.e. in the longitudinal direction).

The terms "upstream" and "downstream" are used herein with reference to the direction of airflow through the device during normal use of the device (i.e. by way of inhalation at the mouthpiece aperture).

The transverse/radial distance between the aerosol generating portion and the wall of the aerosolisation chamber at the distal/downstream end of the aerosol generating portion may be less than 1 mm, for example less than 0.7 mm, or e.g. less than 0.5 mm. The transverse/radial distance between the aerosol generating portion and the wall of the aerosolisation chamber at the distal/downstream end of the aerosol generating portion may be less than 20% of the diameter of aerosolisation chamber, for example less than 17%, or e.g. less than 15%.

A distal (transversely extending) end surface of the aerosol generating portion (i.e. at the extreme downstream end of the aerosol generating portion) may be being substantially planar. In other words, the aerosol generating portion (and thus the liquid transfer element) may have a flattened or truncated distal end. The combination of the planar/flattened distal end and the aerosolisation chamber of constant cross-sectional area may provide a more evenly dispersed spray from the device. For example, such an arrangement may result in a smaller gap between the aerosolisation chamber walls and the aerosol generating portion (e.g. compared with an arrangement having a conical outlet and/or a conical aerosol generating portion), which may reduce the presence of large droplets of liquid in the aerosol discharged from the device.

The distal end of the aerosol generating portion may be spaced from the mouthpiece aperture/end surface (e.g. transversely extending end surface of the mouthpiece) in an upstream longitudinal direction. That is, the distal (downstream) end of the aerosol generating portion/liquid transfer element may be recessed with respect to the end surface of the mouthpiece. For example, the distal end may be spaced (in the longitudinal direction) from the end surface of the mouthpiece or mouthpiece aperture by less than 1.5 mm, or less than 1 mm, or e.g. less than 0.5 mm.

The aerosolisation chamber may be defined by a tube extending upstream and longitudinally from the end surface of the mouthpiece. The device may comprise a retaining element for retaining the liquid transfer element in position with respect to the mouthpiece. The retaining element may comprise a mounting portion extending about the tube so as to mount the retaining element to the tube. The retaining element may comprise a collar projecting from the mounting portion and at least partly circumscribing the liquid transfer element (e.g. the conveying portion) so as to retain the liquid transfer element against movement relative to the mouthpiece. The liquid transfer element (e.g. the conveying portion) may comprise one or more recesses for receipt of a portion of the collar so as to facilitate retaining of the liquid transfer element by the collar.

The aerosol delivery device may comprise a flow passage for fluid flow therethrough. The flow passage may extend generally in the longitudinal direction between (and may fluidly connect) an inlet of the aerosol delivery device and the aerosolisation chamber. In this respect, a user may draw fluid (e.g. air) into and through the flow passage and aerosolisation chamber of the aerosol delivery device by inhaling at the mouthpiece aperture.

The flow passage may comprise one or more deflections. It may comprise a transverse portion proximal the inlet such that there is a deflection between inlet and the transverse portion of the flow passage.

The flow passage may then comprise a generally longitudinal portion downstream of the transverse portion. The longitudinal portion may extend within a spacing between a device housing (which may be integral with the mouthpiece) and a tank (discussed further below) defining the storage chamber. The flow passage may then deflect again (e.g. radially) at the upper surface of the tank within the mouthpiece, through the aerosolisation chamber, towards the mouthpiece aperture.

The flow passage may be a single (annular) flow passage around the tank or it may comprise two branches which split around the tank and re-join within the mouthpiece e.g. proximal the liquid transfer element.

As is mentioned above, the aerosol delivery device may comprise a tank defining the storage chamber for containing the aerosol precursor (which may be e.g. an e-liquid or a flavour liquid). The aerosol precursor may, for example, comprise a flavourant having a menthol, liquorice, chocolate, fruit flavour (including e.g. citrus, cherry etc.), vanilla, spice (e.g. ginger, cinnamon) and/or tobacco flavour.

The aerosol precursor may be stored in the form of a free liquid. Alternatively, a porous body may be disposed within the storage chamber, which may contain the aerosol precursor.

The tank may at least partially define the flow passage. For example, the flow passage may be defined between an outer surface of the tank and an inner surface of a device housing (which may be integral with the mouthpiece)

The aerosol delivery device may comprise an air bleed channel configured to allow the bleeding of air into the storage chamber to replace aerosol precursor that is removed from the storage chamber. The air bleed channel may be in fluid communication with the flow passage, such that (e.g. under certain conditions) air from the flow passage can enter the storage chamber through the air bleed channel.

As will be described further below, the liquid transfer element (i.e. the aerosol generating portion) is configured to generate an aerosol in the aerosolisation chamber. The liquid transfer element does this in such a way that does not use heat to form the aerosol, and therefore is referred to as a "passive" aerosol generator.

The conveying portion may be elongate and generally cylindrical, and may be at least partially enclosed within one or more internal walls of the aerosol delivery device. The one or more internal walls enclosing the conveying portion may form part of the tank defining the storage chamber. In this respect, the tank may at least partly surround (e.g. may fully surround) the conveying portion. That is, the tank may define a conduit through which the conveying portion passes. Thus, the conveying portion may extend generally longitudinally (e.g. centrally) through a portion of the tank (i.e. through the conduit defined by the tank).

The liquid transfer element (i.e. conveying portion) may extend into the storage chamber so as to be in contact with (e.g. at least partially submerged in) the aerosol precursor. Hence, the liquid transfer element may be configured to convey the aerosol precursor from the storage chamber to the aerosolisation chamber via a wicking/capillary action.

The aerosol may be sized to inhibit pulmonary penetration. The aerosol may be formed of particles with a mass median aerodynamic diameter that is greater than or equal to 15 microns, e.g. greater than 30 microns, or greater than 50 microns, or may be greater than 60 microns, or may be greater than 70 microns.

The aerosol may be sized for transmission within at least one of a mammalian oral cavity and a mammalian nasal cavity. The first aerosol may be formed by particles having a maximum mass median aerodynamic diameter that is less than 300 microns, or e.g. less than 200 microns, or less than 100 microns. Such a range of mass median aerodynamic diameter can produce aerosols which are sufficiently small to be entrained in an airflow caused by a user drawing air through the aerosol delivery device and to enter and extend through the oral and or nasal cavity to activate the taste and/or olfactory receptors.

The size of aerosol formed without heating may be typically smaller than that formed by condensation of a vapour.

It is noted that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

The above configuration of the aerosol delivery device may be representative of an activated state of the aerosol delivery device. The aerosol delivery device may additionally be configurable in a deactivated state. In the deactivated state, the liquid transfer element may be isolated from the aerosol precursor. This isolation may, for example, be provided by a plug (e.g. formed of silicone). The plug may be located at an end (i.e. upstream end) of the conduit (defined by the tank) so as to provide a barrier between the aerosol precursor in the storage chamber and the conveying portion of the liquid transfer element. Alternatively, the aerosol delivery device may comprise a duck bill valve, a split valve or diaphragm; or a sheet of foil isolating the liquid transfer element from the first aerosol precursor.

In the deactivated state, the air bleed channel may be sealed by a sealing element. The sealing element may, for example, be in the form of a bung or plug (e.g. a silicone bung or plug). At least a portion of the bung may be received in the air bleed channel when the aerosol delivery device is in the deactivated state, so as to block the passage of airflow through the air bleed channel. The sealing element may alternatively be in the form of a pierceable membrane (e.g. formed of a metal foil) extending across the air bleed channel.

The mouthpiece/device housing of the device may be movable relative to the tank defining the storage chamber. The mouthpiece/device housing may be movable relative to the air bleed channel. In particular, movement of the mouthpiece/device housing may be in the longitudinal direction of the aerosol delivery device.

The mouthpiece may comprise an activation member, which may protrude internally (in an upstream direction) from an internal surface of the mouthpiece. When the mouthpiece/device housing is moved longitudinally in an upstream direction i.e. towards the storage tank, a distal end of the activation member may engage the sealing element so as to move the sealing element (i.e. in the upstream direction) relative to the air bleed channel. This movement of the sealing element may open the air bleed channel, so as to allow airflow therethrough and so as to move the aerosol delivery device to the activated state.

When the sealing element is a bung, the bung may comprise an enlarged end that extends fully across the air bleed channel, and a neck portion that extends only partway across the air bleed channel. Movement of the bung along the air bleed channel by the activation member may cause the enlarged end of the bung to move into the storage chamber such that only the neck portion remains in the air bleed channel. Thus, airflow may be permitted through the air bleed channel between the neck portion and the walls of the air bleed channel.

When the sealing element is a pierceable membrane, the activation member may pierce the pierceable membrane when moved in the upstream direction. To facilitate such piercing, the activation member may be in the form of a blade, or may be pointed.

The movement of the mouthpiece/device housing may also cause longitudinal upstream movement of the liquid transfer element through the conduit defined by the tank. The conveying portion of the liquid transfer element may engage the plug (or duck bill valve, split valve, etc.) so as to disengage the plug from the end of the conduit. Removal of the plug in this way means that the conveying portion comes into contact with the aerosol precursor (i.e. so as to be able to convey the aerosol precursor to the aerosol generating portion of the liquid transfer element).

The above components of the aerosol delivery device may collectively be referred to as an (additive) delivery article or (flavour) pod of the aerosol delivery device.

The aerosol delivery device may further comprise a cartomizer. The (additive) delivery article/(flavour) pod may be engageable with the cartomizer, for example, by way of an interference fit, snap-engagement, bayonet locking arrangement, etc. In other embodiments, the (additive) delivery article/(flavour) pod and cartomizer may be integrally formed.

The device housing may comprise opposing recesses or apertures for engagement with respective lugs provided on the cartomizer to secure the device housing to the cartomizer. There may be two sets of longitudinally spaced lugs and two sets of longitudinally spaced apertures with only the downstream lugs engaged within the upstream apertures when the device is in its deactivated state. Movement of the mouthpiece/device housing cases engagement of the upstream lugs in the upstream apertures and the downstream lugs in the downstream apertures.

The cartomizer may comprise a vaporising chamber and a vapour outlet for fluid flow therethrough. The vapour outlet may be fluidly connected to the flow passage of the additive delivery article/flavour pod. The vapour outlet and vaporising chamber may fluidly connect a cartomizer inlet opening and the inlet of the flow passage. Thus, an airflow may be drawn into and through the cartomizer, and subsequently through the additive delivery article/flavour pod.

The aerosol precursor stored in the storage chamber (and conveyed by the liquid transfer element) may be a first aerosol precursor, and the aerosol formed from the first aerosol precursor may be a first aerosol. The aerosol delivery device (i.e. cartomizer) may comprise a reservoir defined by a container for containing a second aerosol precursor (which may be an e-liquid). The second aerosol precursor may, for example, comprise a base liquid and a physiologically active compound e.g. nicotine. The base liquid may include an aerosol former such as propylene glycol and/or vegetable glycerine.

At least a portion of the container may be translucent. For example, the container may comprise a window to allow a user to visually assess the quantity of second aerosol precursor in the container. The cartomizer may be referred to as a "clearomizer" if it includes a window. The vapour outlet may extend longitudinally through the container, wherein an outlet wall of the vapour outlet may define the inner wall of the container. In this respect, the container may surround the vapour outlet, such that the container may be generally annular.

The aerosol delivery device (i.e. the cartomizer) may comprise a vaporiser. The vaporiser may be located in the vaporising chamber.

The vaporiser may comprise a wick. The vaporiser may further comprise a heater. The wick may comprise a porous material. A portion of the wick may be exposed to fluid flow in the vaporising chamber. The wick may also comprise one or more portions in contact with the second aerosol precursor stored in the reservoir. For example, opposing ends of the wick may protrude into the reservoir and a central portion (between the ends) may extend across the vaporising chamber so as to be exposed to air flow in the vaporising chamber. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the reservoir) to be heated so as to form a vapour and become entrained in fluid/air flowing through the vaporising chamber. This vapour may subsequently cool to form an aerosol in the vapour outlet. This aerosol is hereinafter referred to as the second aerosol. This aerosol generation may be referred to as "active" aerosol generation, because it makes use of heat to generate the aerosol.

This second aerosol may subsequently flow from the vapour outlet to (and through) the flow passage and aerosolisation chamber of the additive delivery article/flavour pod (e.g. when engaged with the cartomizer). Thus, the fluid received through the mouthpiece aperture of the aerosol delivery device may be a combination of the first aerosol and the second aerosol.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The (additive) delivery article ((flavour) pod) and/or cartomizer may be a consumable part of an aerosol delivery system. In this regard, the device may be a termed "a consumable".

### Summary of the Figures

So that the invention may be understood, and so that further aspects and features thereof may be appreciated, embodiments illustrating the principles of the invention will now be discussed in further detail with reference to the accompanying figures, in which:
Figures 1A and 1B is a schematic drawing of an aerosol delivery system according to a first embodiment;
Figures 2A and 2B is a schematic drawing of an aerosol delivery system according to a second embodiment;
Figure 3A is a cross-sectional view of a consumable, according to a third embodiment, in a deactivated state;
Figure 3B is a cross-sectional view of the consumable of Figure 3A in an activated state;
Figure 3C is a cross-sectional schematic view of the flavour pod portion of the consumable of the third embodiment;
Figures 3D and 3E are respective top and perspective views of a mouthpiece of the third embodiment;
Figure 4A is a cross-sectional view of a consumable, according to a fourth embodiment, in a deactivated state;
Figure 4B is a cross-sectional view of a consumable, according to a fourth embodiment, in an activated state;
Figure 4C shows an enlarged view of the mouthpiece portion of the fourth embodiment;
Figure 5A is a cross-sectional view of a consumable that is a variation of the third embodiment, in a deactivated state;
Figure 5B is a cross-sectional view of the consumable of Figure 5A in an activated state;
Figure 5C is a cross-sectional schematic view of the flavour pod portion of the consumable of the variation of the third embodiment;
Figure 6 is a detailed cross-sectional view of the mouthpiece of a consumable that is a variation of the fourth embodiment
Figure 7A is a perspective view of a hinged cap, in a sealed configuration; and
Figure 7B is a perspective view of the hinged cap of Figure 7A in an open configuration.

### Detailed Description

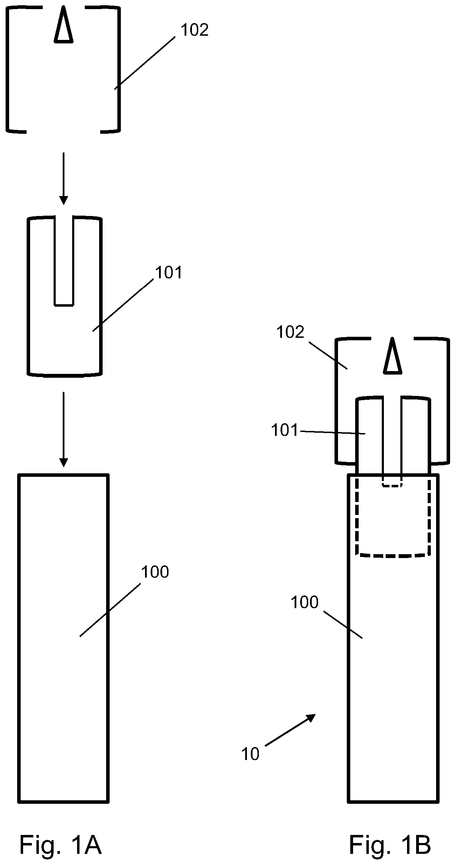

Aspects and embodiments of the present disclosure will now be discussed with reference to the accompanying figures. Further aspects and embodiments will be apparent to those skilled in the art. Referring to figures 1A and 1B, there is shown a schematic view of an aerosol delivery system in the form of a smoking substitute system 10. In this example, the smoking substitute system 10 comprises a cartomizer 101 and an additive delivery article in the form of a flavour pod 102 connected to a base unit 100. In this example, the base unit 100 includes elements of the smoking substitute system 10 such as a battery, an electronic controller, and a pressure transducer (not shown). The cartomizer 101 may engage with the base unit 100 via a push-fit engagement, a screw-thread engagement, or a bayonet fit, for example. A cartomizer may also be referred to as a "pod".

The flavour pod 102 is configured to engage with the cartomizer 101 and thus with the base unit 100. The flavour pod 102 may engage with the cartomizer 101 via a push-fit engagement, a screw-thread engagement, or a bayonet fit, for example. Figure 1B illustrates the cartomizer 101 engaged with the base unit 100, and the flavour pod 102 engaged with the cartomizer 101. As will be appreciated, in this example, the cartomizer 101 and the flavour pod 102 are distinct elements.

As will be appreciated from the following description, in other embodiments the cartomizer 101 and the flavour pod 102 may be combined into a single component that implements the combined functionality of the cartomizer 101 and flavour pod 102. Such a single component may also be referred to as an aerosol delivery device. In other examples, the cartomizer may be absent, with only a flavour pod 102 present.

As is set forth above, reference to a "consumable" component may mean that the component is intended to be used once until exhausted, and then disposed of as waste or returned to a manufacturer for reprocessing.

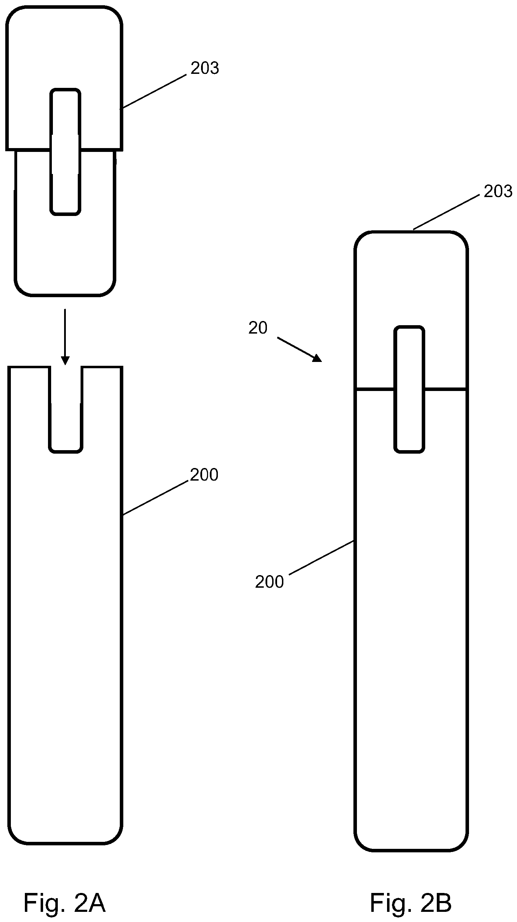

Referring to figures 2A and 2B, there is shown a smoking substitute system 20 comprising a base unit 200 and a consumable 203. The consumable 203 combines the functionality of the cartomizer 201 and the flavour pod 202. In Figure 2A, the consumable 203 and the base unit 200 are shown separated from one another. In Figure 2B, the consumable 203 and the base unit 200 are engaged with each other to form the smoking substitute system 20.

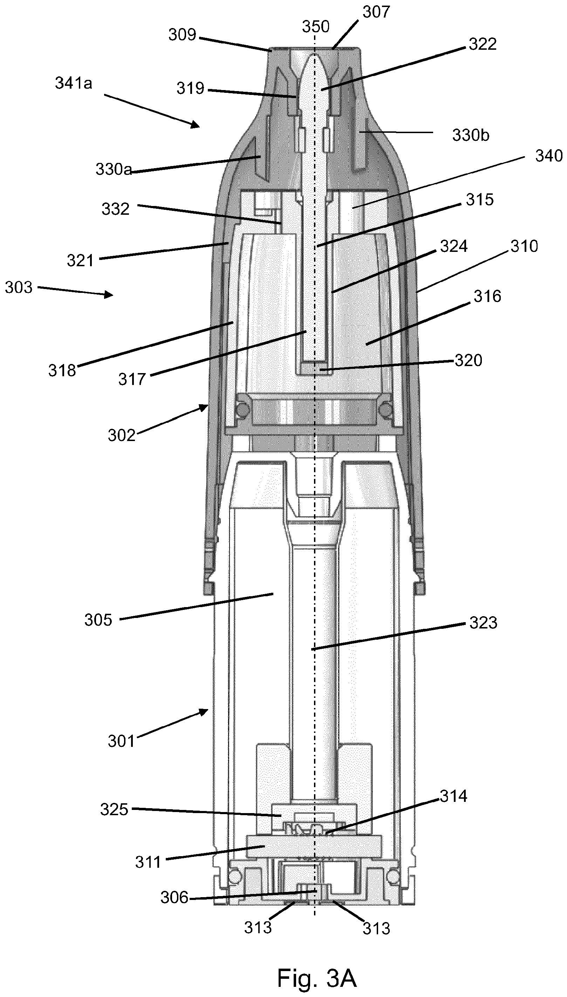

Referring to Figure 3A, there is shown a consumable 303 engagable with a base unit (not shown) via a push-fit engagement. The consumable 303 is shown in a deactivated state. The consumable 303 may be considered to have two portions - a cartomizer portion 301 and flavour pod portion 302 (i.e. additive delivery article), both of which are located within a single consumable component 303 (as in figures 2A and 2B). It should, however, be appreciated that in a variation, the cartomizer portion 301 and flavour pod portion 302 may be separate (but engageable) components.

The consumable 303 includes an upstream cartomizer inlet opening 306 and a downstream mouthpiece aperture 307 (i.e. defining an outlet of the consumable 303) provided in a mouthpiece portion 309 of a terminal element 341. In other examples a plurality of inlets and/or outlets are included. Between, and fluidly connecting, the inlet opening 306 and the mouthpiece aperture 307 there is an airflow path (or passage) comprising (in a downstream flow direction) a vaporising chamber 325 of the cartomizer, a vapour outlet 323 (also of the cartomizer) and a downstream flow passage 321 (which will hereinafter be referred to as the vapour flow passage) of the flavour pod portion 302.

As above, the consumable 303 includes a flavour pod portion 302. The flavour pod portion 302 is configured to generate a first (flavoured) aerosol for output from the mouthpiece aperture 307. The flavour pod portion 302 of the consumable 303 includes a liquid transfer element 315. This liquid transfer element 315 acts as a passive aerosol generator (i.e. an aerosol generator which does not use heat to form the aerosol), and is formed of a porous material. The liquid transfer element 315 comprises a conveying portion 317 and an aerosol generating portion 322, which is located in the vapour flow passage 321. In this example, the aerosol generating portion 322 is a porous nib.

When activated, as discussed in more detail below, a storage chamber 316 (defined by a tank 318) for storing an aerosol precursor (i.e. a liquid comprising a flavourant) is fluidly connected to the liquid transfer element 315. The flavoured aerosol precursor, in this embodiment, is stored in a porous body within the storage chamber 316 (but may be a free-liquid). In the activated state, the liquid transfer element 315 is in contact with the flavoured aerosol precursor stored in the storage chamber 316 by way of contact with the porous body/free liquid.

The liquid transfer element 315 comprises an aerosol generating portion 322 and a conveying portion 317. The aerosol generating portion 322 is located at a downstream end (top of Figure 3A) of the liquid transfer element 315, whilst the conveying portion 317 forms the remainder of the liquid transfer element 315. The conveying portion 317 is elongate and substantially cylindrical. The aerosol generating portion 322 is bulb/bullet-shaped, and comprises a portion which is wider (has a greater radius) than the conveying portion 317. The aerosol generating portion 322 tapers to a tip at a downstream end of the liquid transfer element 315.

The liquid transfer element 315 extends into and through the storage chamber 316, such that the conveying portion 317 is in contact with the contents of the storage chamber 316. In particular, an inner wall of the tank 318 defines a conduit 324, through which the liquid transfer element 315 extends. The liquid transfer element 315 and the conduit 324 are located in a substantially central position within the storage chamber 316 and are substantially parallel to a central longitudinal axis of the consumable 303.

The porous nature of the liquid transfer element 315 means that first (flavoured) aerosol precursor in the storage chamber 316 is drawn into the liquid transfer element 315. As the flavoured aerosol precursor in the liquid transfer element 315 is depleted in use, further flavoured aerosol precursor is drawn from the storage chamber 316 into the liquid transfer element 315 via a wicking action.

Before activation, the storage chamber 316 is fluidly isolated from the liquid transfer element 315. In this example, the isolation is achieved via a plug 320 (preferably formed from silicone) located at one end of a conduit 324 surrounding the liquid transfer element 315. In other examples, the plug may be replaced by any one of: a duck bill valve; a split valve or diaphragm; or a sheet of foil.

The storage chamber 316 further includes an air bleed channel 332, which in the deactivated state is sealed by a sealing element comprising a pierceable membrane (preferably made from foil). A first activation (or piercing) member 330a, which projects inwardly from an inner surface of the mouthpiece portion 309, and may take the form of a blade, pierces the pierceable membrane and opens the air bleed channel 332 when the consumable is moved to the activated state (as is discussed in more detail below).

The mouthpiece portion 309 of the terminal element 341 further comprises a second activation member 330b. Both activation members 330a, 330b extend symmetrically (i.e. evenly spaced) on either side of the mouthpiece aperture 209 and either side of the liquid transfer element 315. The two activation members extend generally longitudinally parallel to the liquid transfer element and parallel to the longitudinal axis 350 of the device.

In the first orientation of the terminal element 341 shown in Figure 3A, the first activation member 330a is longitudinally aligned with the sealing element. The second activation member 330b is longitudinally aligned with a filling port 340 which is blocked by a plug (not shown).

The device may alternatively be assembled with the terminal element 240 rotated through 180 degrees so that the second activation member 330b is longitudinally aligned with the sealing element and the first activation member 330a is longitudinally aligned with the filling port 340.

The aerosol generating portion 322 of the liquid transfer element 315 is located within the vapour flow passage 321 that extends through the flavour pod portion 302. The aerosol generating portion 322, by occupying a portion of the vapour flow passage 321, constricts or narrows the vapour flow passage 321. This constricted or narrowed portion of the vapour flow passage 321 defines an aerosolisation chamber 319 of the consumable 303. The aerosolisation chamber 319, which is adjacent the aerosol generating portion 322, is the narrowest portion of the vapour flow passage 321. The constriction of the vapour flow passage 321 at the aerosolisation chamber 319 results in increased air velocity and a corresponding reduction in air pressure of the air flowing therethrough and thus may be referred to as a Venturi aperture. The aerosolisation chamber 319 is generally toroidal in shape (extending circumferentially about the aerosol generating portion 322), but this toroidal shape may include one or more interruptions where supports extend inwardly to contact the aerosol generating portion 322 and to support the aerosol generating portion 322 within the aerosolisation chamber 319.

The cartomizer portion 301 of the consumable 303 includes a reservoir 305 (defined by a container) for storing a second (e-liquid) aerosol precursor (which may contain nicotine). A wick 311 extends into the reservoir so as to be in contact with (i.e. partially submerged in) the e-liquid aerosol precursor. The wick 311 is formed from a porous wicking material (e.g. a polymer) that draws the e-liquid aerosol precursor from the reservoir 305 into a central region of the wick 311 that is located in the vaporising chamber 325.

A heater 314 is a configured to heat the central region of the wick 311. The heater 314 includes a resistive heating filament that is coiled around the central region of the wick 311. The wick 311 and the heater 314 generally define a vaporiser, and together with the reservoir 305 act as an active aerosol generator. The vaporiser (i.e. wick 311 and heater 314) and aerosol generating portion 322 are both at least partially located within the airflow passage, with the aerosol generating portion 322 being downstream of the vaporiser.

So that the consumable 303 may be supplied with electrical power for activation of the heater 314, the consumable 303 includes a pair of consumable electrical contacts 313. The consumable electrical contacts 313 are configured for electrical connection to a corresponding pair of electrical supply contacts in the base unit (not shown). The consumable electrical contacts 313 are electrically connected to the electrical supply contacts (not shown) when the consumable 303 is engaged with the base unit. The base unit includes an electrical power source, for example a battery.

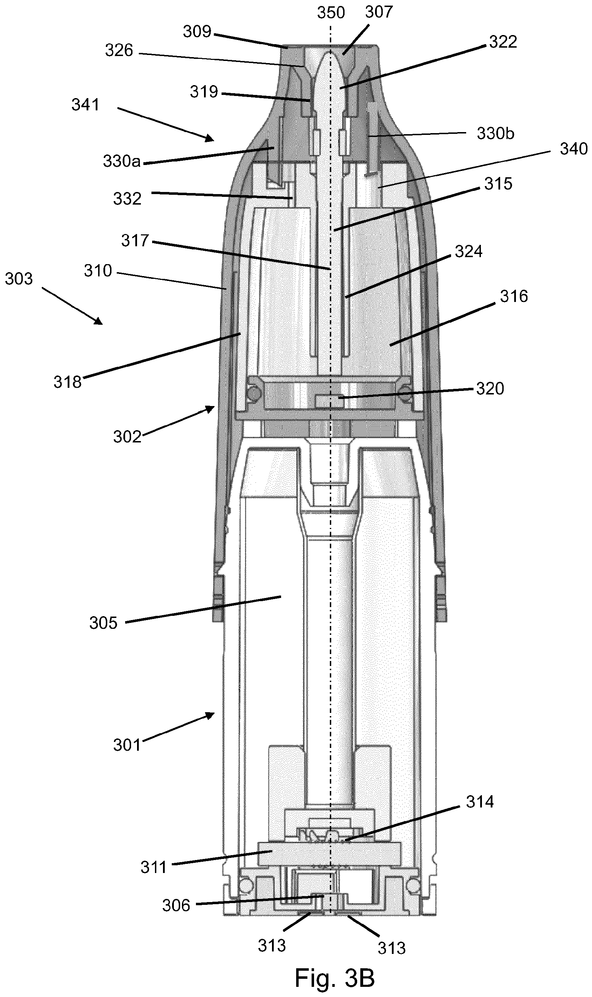

Figure 3B shows the consumable 303 of Figure 3A in an activated state. To transition from the deactivated state to the activated state, terminal element 341 is moved along the central longitudinal axis 350 in an upstream direction towards cartomizer portion 301. The mouthpiece portion 309 of the terminal element 341 is fixed by a collar 308 to the conveying portion 317 of the liquid transfer element 315 and therefore liquid transfer element 315 moves with the mouthpiece 309. The mouthpiece 309 and liquid transfer element 315 are moved relative to the tank 316.

When the mouthpiece 309 is moved upstream, first activation/piercing member 330a contacts and pierces the sealing element in the form of a pierceable membrane extending across the air bleed channel 332 thereby fluidly connecting the vapour flow passage 321 the storage chamber 316. This allows air from the vapour flow passage 321 to enter the storage chamber 316 as aerosol precursor is removed from the storage chamber 316 by the liquid transfer element 315.

In addition to piercing of the membrane by the first activation member 330, liquid transfer element 315 pushes on, and moves, plug 320 out of the conduit 324 which then allows liquid transfer element 315 to come into contact with the flavoured aerosol precursor stored in the storage chamber 316. The plug 320 may then be unconstrained within the storage chamber, or may be pushed by liquid transfer element 315 into a holding location.

As the terminal element 341 is moved upstream, the second activation member is received within the plug (not shown) sealing the filling port 340.

Of course, when the device is assembled with the terminal element 341 in the second orientation, in the activated state, the second activation member 330b pierces the sealing element membrane and the first activation member 330a is received within the filling port 340.

Once activated, and in use, a user draws (or "sucks", "pulls", or "puffs") on the mouthpiece portion 309 of the consumable 303, which causes a drop in air pressure at the mouthpiece aperture 307, thereby generating air flow through the inlet opening 306, along the airflow path including the vapour passage, out of the mouthpiece aperture 307 and into the user's mouth.

When the heater 314 is activated by passing an electric current through the heating filament in response to the user drawing on the mouthpiece portion 309 (the drawing of air may be detected by a pressure transducer), the e-liquid located in the wick 311 adjacent to the heating filament is heated and vaporised to form a vapour in the vaporising chamber 325. The vapour condenses to form the e-liquid aerosol within the vapour outlet 323. The e-liquid aerosol is entrained in an airflow along the vapour flow passage 321 to the mouthpiece aperture 307 for inhalation by the user when the user draws on the mouthpiece portion 309.

The base unit supplies electrical current to the consumable electrical contacts 113. This causes an electric current flow through the heating filament of the heater 314 and the heating filament heats up. As described, the heating of the heating filament causes vaporisation of the e-liquid in the wick 311 to form the e-liquid aerosol.

As the air flows through the vapour flow passage 321, it encounters the aerosol generating portion 322. The constriction of the vapour flow passage 321, at the aerosolisation chamber 319, results in an increase in air velocity and corresponding decrease in air pressure in the airflow in the vicinity of the porous aerosol generating portion 322. The corresponding low pressure and high air velocity region causes the generation of the flavoured aerosol from the porous surface of the aerosol generating portion 322 of the liquid transfer element 315. The flavoured aerosol becomes entrained in the airflow and ultimately is output from the mouthpiece aperture 307 of the consumable 303 and into the user's mouth.

The flavoured aerosol is sized to inhibit pulmonary penetration. The flavoured aerosol is formed of particles with a mass median aerodynamic diameter that is greater than 70 microns. The flavoured aerosol is sized for transmission within at least one of a mammalian oral cavity and a mammalian nasal cavity. The flavoured aerosol is formed by particles having a maximum mass median aerodynamic diameter that is less than 100 microns. Such a range of mass median aerodynamic diameter will produce aerosols which are sufficiently small to be entrained in an airflow caused by a user drawing air through the device and to enter and extend through the oral and or nasal cavity to activate the taste and/or olfactory receptors.

The e-liquid aerosol generated is sized for pulmonary penetration (i.e. to deliver an active ingredient such as nicotine to the user's lungs). The e-liquid aerosol is formed of particles having a mass median aerodynamic diameter of less than 1 micron. Such sized aerosols tend to penetrate into a human user's pulmonary system, with smaller aerosols generally penetrating the lungs more easily. The e-liquid aerosol may also be referred to as a vapour.

The size of aerosol formed without heating is typically smaller than that formed by condensation of a vapour.

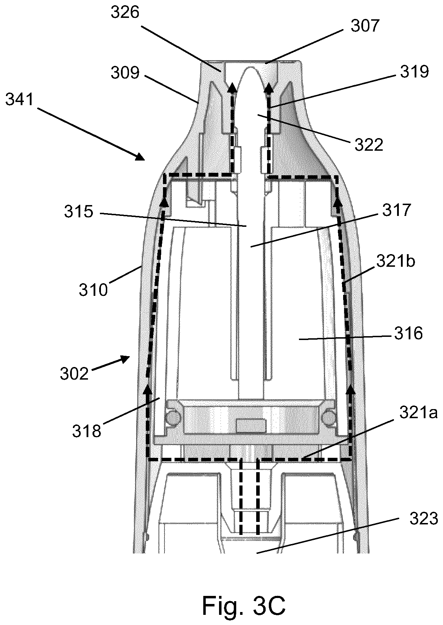

Figure 3C illustrates the flow of vapour through the flavour pod portion 302 of figures 3A and 3B (although the second activation member 330b is omitted). The flavour pod portion 302 is shown in the activated state with the terminal housing in its first orientation. The cartomizer is not shown, but it should be appreciated that the flavour pod portions 302 is engaged with the cartomizer 301 of figures 3A and 3B. In other embodiments, however, the consumable 303 may not comprise a cartomizer portion, and may provide only flavour to the user.

As is provided above, the flavour pod portion 302 comprises an upstream (i.e. upstream with respect to flow of air in use) vapour passage inlet 304 (in fluid communication with the vapour outlet 323) and a downstream (i.e. downstream with respect to flow of air in use) outlet in the form of a mouthpiece aperture 307. Between, and fluidly connecting the vapour passage inlet 304 and the mouthpiece aperture 307, is a vapour flow passage 321.

The vapour flow passage 321 comprises a transverse portion 321a. The airflow path through the device deflects at the vapour passage inlet 304 i.e. there is a deflection between the vapour outlet 323 and the transverse portion 321a of the vapour flow passage 321.

The vapour flow passage 321 then deflects again from the transverse portion 321a to a longitudinal portion 321b which extends generally longitudinally between a device housing 310 (which forms part of the terminal element 341 and which is integral with the mouthpiece portion 309) and the tank 318. The vapour flow passage deflects again at the upper surface of the tank 318 within the mouthpiece portion 309, through the aerosolisation chamber 319, towards the mouthpiece aperture 307. Within the mouthpiece portion 309, flow around the first and second activation members 330a, 330b is symmetrical.

The vapour flow passage 321 may be a single (annular) flow passage around the tank 318 or it may comprise two branches which split around the tank 318 and re-join within the mouthpiece portion 309 proximal the liquid transfer element 315.

A transition surface 326, between the aerosolisation chamber 319 and the mouthpiece aperture 307 flares outwardly in the downstream direction, such that a diameter of the mouthpiece aperture 307 is greater than a diameter of the aerosolisation chamber 319.

In use, when a user draws on the mouthpiece portion 309, air flow is generated through the air flow path through the device. Air (comprising the e-liquid aerosol from the cartomizer portion 301 as explained above with respect to Figure 3A) flows through the vapour outlet 323 and into the vapour passage 321. Further downstream, as air flows past the aerosol generating portion 322 in the aerosolisation chamber 319, the velocity of the air increases, resulting in a drop in air pressure. As a result, the flavoured aerosol precursor held in the aerosol generating portion 322 becomes entrained in the air so as to form the flavoured aerosol. The flavoured aerosol has the particle size and other properties described above with respect to Figure 3A.

As the flavoured aerosol precursor becomes entrained within the air, the liquid transfer element 315 transfers further flavoured aerosol precursor from the storage chamber 316 to the aerosol generating portion 322. More specifically, the liquid transfer element wicks the flavoured aerosol precursor from the storage chamber 316 to the aerosol generating portion 322.

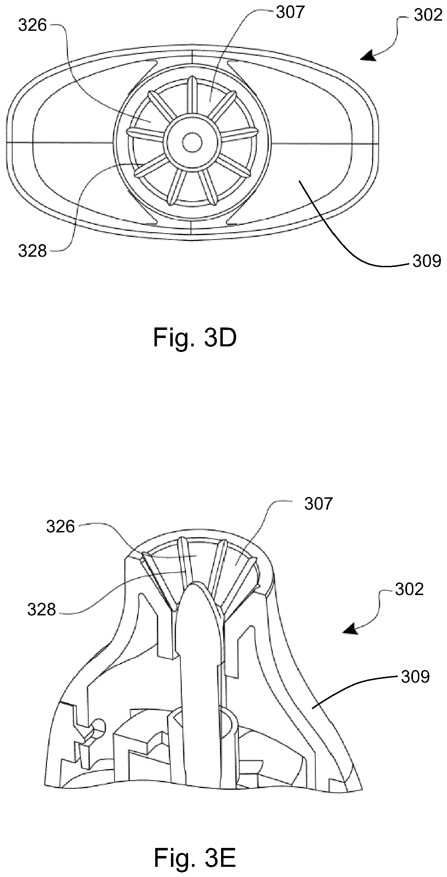

Figures 3D and 3E show further views of the flavour pod portion 302 which highlight features of the mouthpiece portion 309. Many of the reference numerals of Figure 3C are omitted from figures 3D and 3E for clarity. The activation members are also omitted.

An uneven inner (transition) surface 326 is located between the mouthpiece aperture 307 and the aerosolisation chamber 319. In the present example, the inner surface 326 has the form of a substantially frustoconical surface, but includes grooves or channels 328 to make the inner surface 326 somewhat uneven. In other examples, the inner surface 326 may have another form (for example, the form a substantially cylindrical surface), and may include any type of protrusion or groove to make the inner surface uneven.

The inner surface 326 is angled with respect to an axial direction (i.e. relative to a central axis extending from a base of the consumable to the mouthpiece) such that the diameter of the passage 321 proximate the mouthpiece aperture 307 increases in the downstream direction. The inner surface 326 is downstream of the aerosolisation chamber 319 of the vapour flow passage 321.

The grooves 328 are generally V-shaped in cross-sectional profile, and extend in the axial direction for the full length of the inner surface 326. Each groove 328 is formed from a pair of surfaces angled at between 30 and 90 degrees (e.g. 60 degrees) relative to each other. The grooves 328 have a depth (measured normal to the inner surface 326) of at least 0.2 mm (e.g. at least 0.4 mm). The grooves 328 have a depth of less than 0.8 mm (e.g. less than 0.6 mm). The grooves have a depth of substantially 0.5 mm. The inner surface 326 comprises 9 grooves 328, but may comprise more or less grooves.

The grooves 328 are spaced apart from each other by substantially 1 mm at the downstream end of the inner surface 326. In other examples, the spacing at the downstream end of grooves or protrusions may be selected such that it is equal to or less than the mass median diameter (as described above) of particles in the flavoured aerosol.

The inner surface 326 comprises a smooth polished surface between the grooves 328. Polishing the surface in this way may provide improved aerodynamic properties. However, in other examples, the inner surface 426 may be textured. In such examples, the texture of the surface may provide the uneven surface, and no grooves may be required.

In use, the uneven nature of the inner surface 326 may make it easier for droplets to form on the inner surface 326, preventing large droplets from entering the user's mouth. The grooves 328 may help to channel the large droplets back into the consumable.

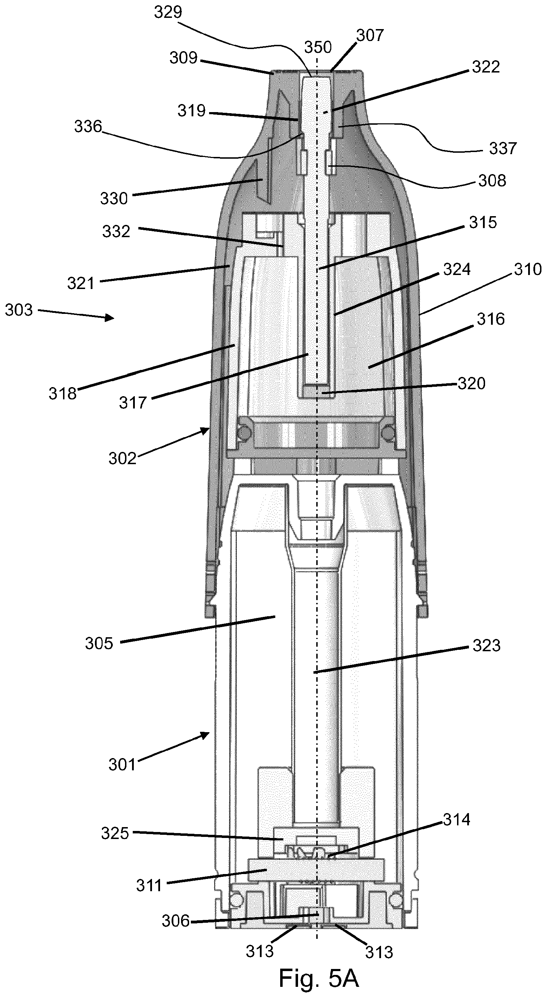

Figures 5A to 5C illustrate a variation of the embodiment shown in figures 3A to 3E and thus the same reference numerals have been used for corresponding features. Referring to Figure 5A, there is shown a consumable 303 engagable with a base unit (not shown) via a push-fit engagement. The consumable 303 is shown in a deactivated state. The consumable 303 may be considered to have two portions - a cartomizer portion 301 and flavour pod portion 302 (i.e. additive delivery article), both of which are located within a single consumable component 303 (as in figures 2A and 2B). It should, however, be appreciated that in a variation, the cartomizer portion 301 and flavour pod portion 302 may be separate (but engageable) components.

The consumable 303 includes an upstream cartomizer inlet opening 306 and a downstream mouthpiece aperture 307 (i.e. defining an outlet of the consumable 303). In other examples a plurality of inlets and/or outlets are included. Between, and fluidly connecting, the inlet opening 306 and the mouthpiece aperture 307 there is an airflow passage comprising (in a downstream flow direction) a vaporising chamber 325 of the cartomizer, a vapour outlet 323 (also of the cartomizer), a downstream flow passage 321 (hereinafter referred to as the vapour flow passage) of the flavour pod portion 302, and an aerosolisation chamber 319 (also of the flavour pod portion 302). The mouthpiece aperture 307 is located at the mouthpiece 309 (or mouthpiece portion) of the consumable 303.

The aerosolisation chamber 319 extends longitudinally in an upstream direction from the mouthpiece aperture 307. The aerosolisation chamber 319 is cylindrical (having a circular cross-sectional shape) such that a transverse cross-sectional area of the aerosolisation chamber 319 is uniform along a longitudinal length of the aerosolisation chamber 319. The aerosolisation chamber 319 is defined by a tube 337 that extend longitudinally from a longitudinal end surface of the device.

The consumable 303 includes a flavour pod portion 302. The flavour pod portion 302 is configured to generate a first (flavoured) aerosol for output from the mouthpiece aperture 307. The flavour pod portion 302 of the consumable 303 includes a liquid transfer element 315. This liquid transfer element 315 acts as a passive aerosol generator (i.e. an aerosol generator which does not use heat to form the aerosol), and is formed of a porous material. The liquid transfer element 315 comprises a conveying portion 317 and an aerosol generating portion 322, which is located in aerosolisation chamber 319. In this example, the aerosol generating portion 322 is a porous nib.

When activated, as discussed in more detail below, a storage chamber 316 (defined by a tank 318) for storing a first aerosol precursor (i.e. a liquid comprising a flavourant) is fluidly connected to the liquid transfer element 315. The flavoured aerosol precursor, in this embodiment, is stored in a porous body within the storage chamber 316 (but may be a free-liquid). In the activated state, the liquid transfer element 315 is in contact with the flavoured aerosol precursor stored in the storage chamber 316 by way of contact with the porous body/free liquid.

The liquid transfer element 315 comprises an aerosol generating portion 322 and a conveying portion 317. The aerosol generating portion 322 is located at a downstream end (top of Figure 5A) of the liquid transfer element 315, whilst the conveying portion 317 forms the remainder of the liquid transfer element 315. The conveying portion 317 is elongate and substantially cylindrical.

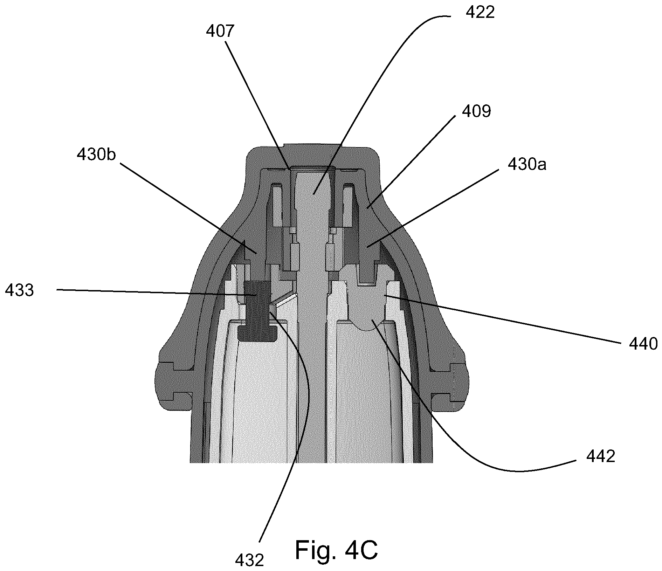

A distal (i.e. downstream) transversely extending end surface 329 of the aerosol generating portion 322 is planar, such that the aerosol generating portion 322 has a somewhat truncated bulb or bullet-shape. The aerosol generating portion 322 is wider (has a greater radius) than the conveying portion 317. As is apparent from Figure 4C (which will be discussed further below) the greater radius of the aerosol generating portion 322 means that a transverse transition surface 336, in the form of a step, is defined between the downstream end of the conveying portion 317 and the upstream end of the aerosol generating portion 322.

The liquid transfer element 315 extends into and through the storage chamber 316, such that the conveying portion 317 is in contact with the contents of the storage chamber 316. In particular, an inner wall of the tank 318 defines a conduit 324, through which the liquid transfer element 315 extends. The liquid transfer element 315 and the conduit 324 are located in a substantially central position within the storage chamber 316 and are substantially parallel to a central longitudinal axis of the consumable 303.

The porous nature of the liquid transfer element 315 means that first (flavoured) aerosol precursor in the storage chamber 316 is drawn into the liquid transfer element 315. As the flavoured aerosol precursor in the liquid transfer element 315 is depleted in use, further flavoured aerosol precursor is drawn from the storage chamber 316 into the liquid transfer element 315 via a wicking action.

Before activation, the storage chamber 316 is fluidly isolated from the liquid transfer element 315. In this example, the isolation is achieved via a plug 320 (preferably formed from silicone) located at one end of a conduit 324 surrounding the liquid transfer element 315. In other examples, the plug may be replaced by any one of: a duck bill valve; a split valve or diaphragm; or a sheet of foil.

The storage chamber 316 further includes an air bleed channel 332, which in the deactivated state is sealed by a sealing element comprising a pierceable membrane (preferably made from foil). Activation (or piercing) member 330, which projects inwardly from the mouthpiece 309, and may take the form of a blade, pierces the pierceable membrane and opens the air bleed channel 332 when the consumable is moved to the activated state (as is discussed in more detail below).

The liquid transfer element 315 (i.e. the aerosol generating portion 322) is located within the aerosolisation chamber 319, such that an airflow path is defined between the liquid transfer element 315 an inner surface of the tube 337 defining the aerosolisation chamber 319. This airflow path comprises a constricted region which is defined by the aerosol generating portion 322. This constriction results in increased air velocity and a corresponding reduction in air pressure of the air flowing along the airflow path and thus the constricted region may be referred to as a Venturi aperture. Although not shown, in some embodiments, one or more supports may extend inwardly from the wall defining the aerosolisation chamber 319 (e.g. tube 337) to contact the aerosol generating portion 322 and to support the aerosol generating portion 322 within the aerosolisation chamber 319.

The cartomizer portion 301 of the consumable 303 includes a reservoir 305 (defined by a container) for storing a second (e-liquid) aerosol precursor (which may contain nicotine). A wick 311 extends into the reservoir so as to be in contact with (i.e. partially submerged in) the e-liquid aerosol precursor. The wick 311 is formed from a porous wicking material (e.g. a polymer) that draws the e-liquid aerosol precursor from the reservoir 305 into a central region of the wick 311 that is located in the vaporising chamber 325.

A heater 314 is a configured to heat the central region of the wick 311. The heater 314 includes a resistive heating filament that is coiled around the central region of the wick 311. The wick 311 and the heater 314 generally define a vaporiser, and together with the reservoir 305 act as an active aerosol generator. The vaporiser (i.e. wick 311 and heater 314) and aerosol generating portion 322 are both at least partially located within the airflow passage, with the aerosol generating portion 322 being downstream of the vaporiser.

So that the consumable 303 may be supplied with electrical power for activation of the heater 314, the consumable 303 includes a pair of consumable electrical contacts 313. The consumable electrical contacts 313 are configured for electrical connection to a corresponding pair of electrical supply contacts in the base unit (not shown). The consumable electrical contacts 313 are electrically connected to the electrical supply contacts (not shown) when the consumable 303 is engaged with the base unit. The base unit includes an electrical power source, for example a battery.

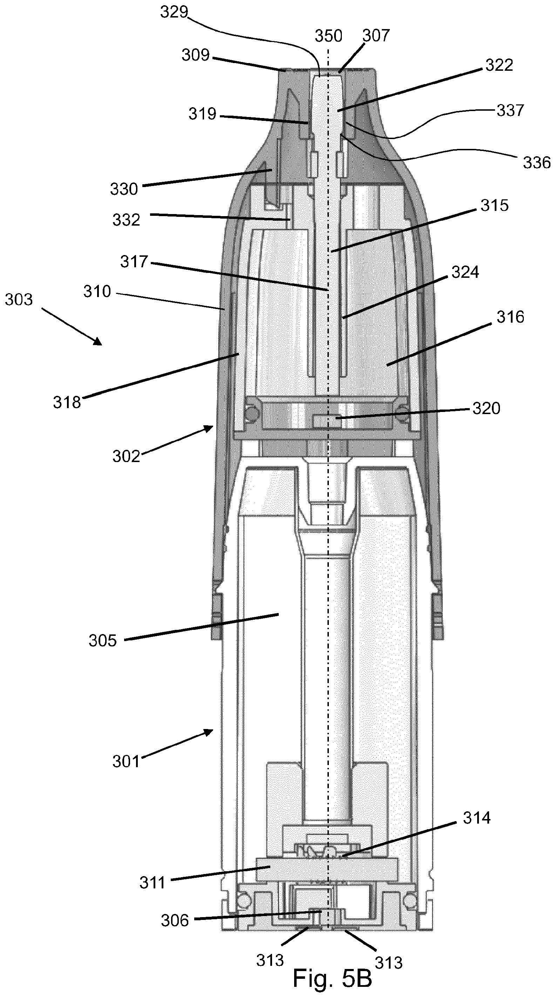

Figure 5B shows the consumable 303 of Figure 5A in an activated state. To transition from the deactivated state to the activated state, mouthpiece 309 is moved along a central longitudinal axis 350 in an upstream direction towards cartomizer portion 301. The mouthpiece 309 is fixed by a collar 308 to the conveying portion 317 of the liquid transfer element 315 and therefore liquid transfer element 315 moves with the mouthpiece 309. The mouthpiece 309 and liquid transfer element 315 are moved relative to the tank 316.

When the mouthpiece 309 is moved upstream, activation/piercing member 330 contacts and pierces a sealing element in the form of a pierceable membrane extending across the air bleed channel 332 thereby fluidly connecting the vapour flow passage 321 the storage chamber 316. This allows air from the vapour flow passage 321 to enter the storage chamber 316 as aerosol precursor is removed from the storage chamber 316 by the liquid transfer element 315.

In addition to piercing of the membrane by the piercing member 330, liquid transfer element 315 pushes on, and moves, plug 320 out of the conduit 324 which then allows liquid transfer element 315 to come into contact with the flavoured aerosol precursor stored in the storage chamber 316. The plug 320 may then be unconstrained within the storage chamber, or may be pushed by liquid transfer element 315 into a holding location.

Once activated, and in use, a user draws (or "sucks", "pulls", or "puffs") on the mouthpiece 309 of the consumable 303, which causes a drop in air pressure at the mouthpiece aperture 307, thereby generating air flow through the inlet opening 306, along the airflow passage, out of the mouthpiece aperture 307 and into the user's mouth.

When the heater 314 is activated by passing an electric current through the heating filament in response to the user drawing on the mouthpiece 309 (the drawing of air may be detected by a pressure transducer), the e-liquid located in the wick 311 adjacent to the heating filament is heated and vaporised to form a vapour in the vaporising chamber 325. The vapour condenses to form the e-liquid aerosol within the vapour outlet 323. The e-liquid aerosol is entrained in an airflow along the vapour flow passage 321, through the aerosolisation chamber 319, to the mouthpiece aperture 307 for inhalation by the user when the user draws on the mouthpiece 309.

The base unit supplies electrical current to the consumable electrical contacts 113. This causes an electric current flow through the heating filament of the heater 314 and the heating filament heats up. As described, the heating of the heating filament causes vaporisation of the e-liquid in the wick 311 to form the e-liquid aerosol.

As the air flows through the aerosolisation chamber 319, the constricted region of the air path results in an increase in air velocity and corresponding decrease in air pressure in the airflow in the vicinity of the porous aerosol generating portion 322. The corresponding low pressure and high air velocity region causes the generation of the flavoured aerosol from the porous surface of the aerosol generating portion 322 of the liquid transfer element 315. The flavoured aerosol becomes entrained in the airflow and ultimately is output from the mouthpiece aperture 307 of the consumable 303 and into the user's mouth.

The flavoured aerosol is sized to inhibit pulmonary penetration. The flavoured aerosol is formed of particles with a mass median aerodynamic diameter that is greater than 70 microns. The flavoured aerosol is sized for transmission within at least one of a mammalian oral cavity and a mammalian nasal cavity. The flavoured aerosol is formed by particles having a maximum mass median aerodynamic diameter that is less than 100 microns. Such a range of mass median aerodynamic diameter will produce aerosols which are sufficiently small to be entrained in an airflow caused by a user drawing air through the device and to enter and extend through the oral and or nasal cavity to activate the taste and/or olfactory receptors.

The e-liquid aerosol generated is sized for pulmonary penetration (i.e. to deliver an active ingredient such as nicotine to the user's lungs). The e-liquid aerosol is formed of particles having a mass median aerodynamic diameter of less than 1 micron. Such sized aerosols tend to penetrate into a human user's pulmonary system, with smaller aerosols generally penetrating the lungs more easily. The e-liquid aerosol may also be referred to as a vapour.

The size of aerosol formed without heating is typically smaller than that formed by condensation of a vapour.

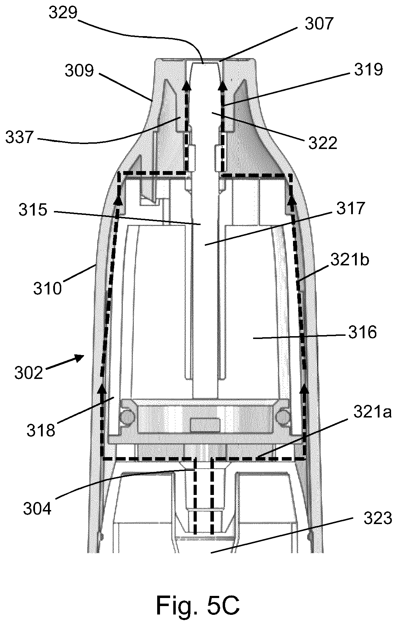

Figure 5C illustrates the flow of vapour through the flavour pod portion 302 of figures 5A and 5B. The flavour pod portion 302 is shown in the activated state. The cartomizer is not shown, but it should be appreciated that the flavour pod portions 302 is engaged with the cartomizer 301 of figures 5A and 5B. In other embodiments, however, the consumable 303 may not comprise a cartomizer portion, and may provide only flavour to the user.

As is provided above, the flavour pod portion 302 comprises an upstream (i.e. upstream with respect to flow of air in use) vapour passage inlet 304 (in fluid communication with the vapour outlet 323) and a downstream (i.e. downstream with respect to flow of air in use) outlet in the form of a mouthpiece aperture 307. Between, and fluidly connecting the vapour passage inlet 304 and the mouthpiece aperture 307, is a vapour flow passage 321 and aerosolisation chamber 319.

The vapour flow passage 321 comprises a transverse portion 321a. The airflow path through the device deflects at the vapour passage inlet 304 i.e. there is a deflection between the vapour outlet 323 and the transverse portion 321a of the vapour flow passage 321.

The vapour flow passage 321 then deflects again from the transverse portion 321a to a longitudinal portion 321b which extends generally longitudinally between a device housing 310 (which is integral with the mouthpiece 309) and the tank 318. The vapour flow passage 321 deflects again at the upper surface of the tank 318 within the mouthpiece 309 such that airflow/vapour is directed through the aerosolisation chamber 319, towards the mouthpiece aperture 307.

The vapour flow passage 321 may be a single (annular) flow passage around the tank 318 or it may comprises two braches which split around the tank 318 and re-join within the mouthpiece 309 proximal the liquid transfer element 315.

In use, when a user draws on the mouthpiece 309, air flow is generated through the air flow passage through the device. Air (comprising the e-liquid aerosol from the cartomizer portion 301 as explained above with respect to Figure 5A) flows through the vapour outlet 323 and into the vapour passage 321. Further downstream, as air flows past the aerosol generating portion 322 in the aerosolisation chamber 319, the velocity of the air increases, resulting in a drop in air pressure. As a result, the flavoured aerosol precursor held in the aerosol generating portion 322 becomes entrained in the air so as to form the flavoured aerosol. The flavoured aerosol has the particle size and other properties described above with respect to Figure 5A.

As the flavoured aerosol precursor becomes entrained within the air, the liquid transfer element 315 transfers further flavoured aerosol precursor from the storage chamber 316 to the aerosol generating portion 322. More specifically, the liquid transfer element wicks the flavoured aerosol precursor from the storage chamber 316 to the aerosol generating portion 322.

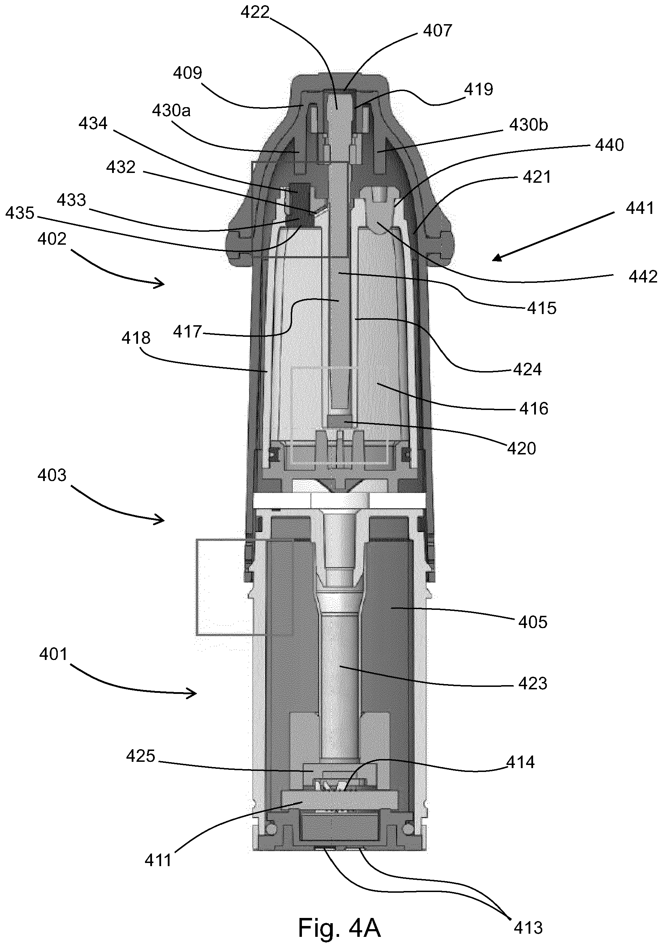

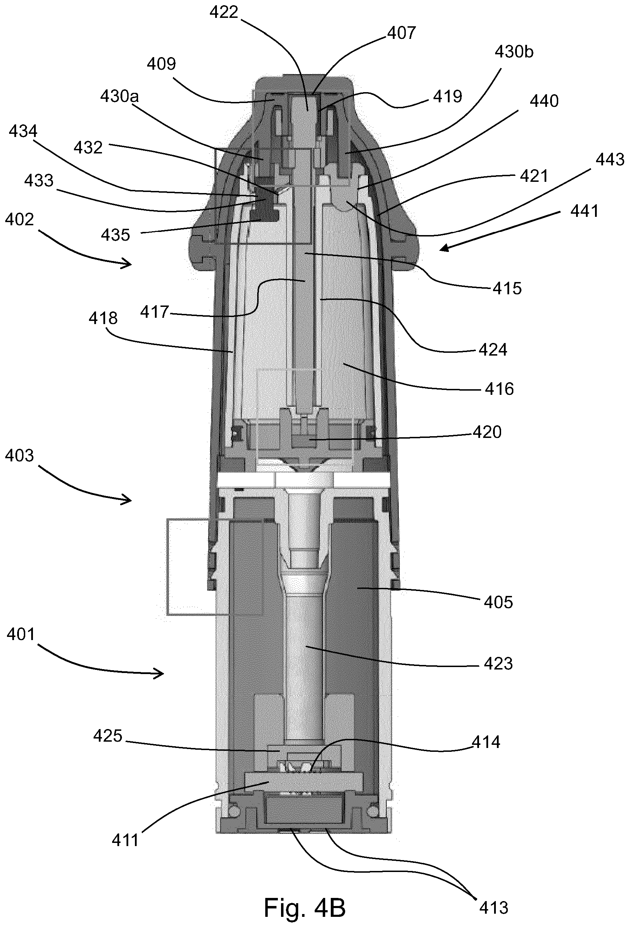

Figures 4A, 4B and 4C illustrate a further embodiment of a consumable 404 of an aerosol delivery device. This embodiment includes many of the same features of the embodiment described above and shown in Figures 3A-3E and Figures 5A-5C and, for that reason, corresponding reference numerals have been used (albeit with a unit increase of the first digit to represent the further embodiment). The description of those features have not been repeated here. Figure 4A shows the consumable 404 in a deactivated state (with the terminal element 341 in its first orientation) and Figure 4B shows the consumable 404 in an activated state (with the terminal element 341 in its first orientation). Figure 4C shows an enlarged view of the mouthpiece portion 409 in the activated state with the terminal element in its second orientation - i.e. with the terminal housing 441 rotated by 180 degrees.

Unlike the previously described embodiment, the presently illustrated embodiment comprises a different mechanism for opening the air bleed channel 432 upon activation of the consumable 403. In this embodiment, when in the deactivated state (Figure 4A) the air bleed channel 432 is obstructed by a silicone bung 433 sealing element received in the air bleed channel 432. In particular, a body 434 of the bung 433 is received in the air bleed channel 432, but does not fully obstruct the air bleed channel 432. That is, a portion of the air bleed channel 432 remains unobstructed by the body 434 of the bung 433. However, an enlarged head 435 of the bung 433, which is located in the storage chamber 416, extends fully across the entrance to the air bleed channel 432 so as to obstruct the channel 432.

When the terminal element 441/mouthpiece portion 409 (alternatively referred to as the mouthpiece 409) is moved in the upstream longitudinal direction to activate the consumable 403, an elongate first activation member 430a (extending inwardly from an inner surface of the mouthpiece portion 409) engages the body 434 of the bung 433 and pushes the bung 433 in the upstream direction (see Figure 4B). This moves the enlarged head 435 of the bung 433 away from the entrance of the air bleed channel 432 such that the head 435 no longer obstructs the air bleed channel 432. This allows air to pass from the vapour flow passage 421 and into the storage chamber 416 (which, in turn, allows for flow of the flavoured aerosol precursor from the storage chamber 416).

It should be noted that the bung sealing element shown in Figures 4A and 4B could be used in place of the pierceable membrane in Figures 3A and 3B and vice versa.

The second activation member 430b is received within a bore provided in a bung 442 sealing the filling port 440.

Of course, when the device is assembled with the terminal element 441 in its second orientation, the first activation member 430a is received within the bore provided in the bung 442 sealing the filling port 440 and the second activation member 430b moves the silicone bung 433 within the air bleed channel 432 as shown in Figure 4C.

The aerosol generating portion 422 of the liquid transfer element shown in the Figures 4A and 4B has a flattened (or planar) upper (downstream) surface. Such a liquid transfer element could be used in the embodiment shown in Figure 3A and 3B.

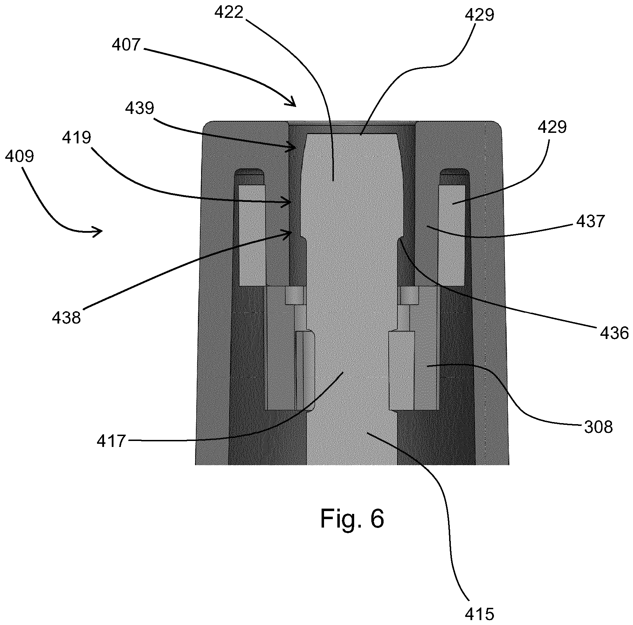

Figure 6 provides a detailed view of a mouthpiece 409 that is similar to that shown in Figures 4A to 4C, albeit having a slightly modified shape. Given the similarity, the same references numerals have been used for corresponding features.

As is apparent from Figure 6, the conveying portion 417 has a smaller cross-sectional area than the aerosol generating portion 422 of the liquid transfer element 415. As a result, a step (from the conveying portion 417 to the aerosol generating portion 422) is defined by a radial transition surface 436. The transition surface 436 has a concave profile such that there is a smooth transition between the conveying portion 417 and the transition surface 436 and a sharp/hard edge between the transition surface 436 and the aerosol generating portion 422.

This edge is an upstream leading edge of the aerosol generating portion 422 and defines an upstream end of a constricted region 438 of an airflow path between the aerosol generating portion 422 and the tube 437 defining the aerosolisation chamber 419. Downstream of this constricted region 438 is an expansion region 439. In this expansion region 329 the airflow path gradually increases in cross-sectional area in the downstream direction. This a result of the aerosol generating portion 422 having a slight inward taper towards the planar distal end surface 429 and the cylindrical shape of the aerosolisation chamber 419.

Also shown in more detail in Figure 6 is the collar 408, which partially circumscribes the liquid transfer element 415. The collar 408 is connected to a mounting portion 440, which surrounds the tube 437 so as to mount the collar 408 to the tube 437. In this way, the liquid transfer element 415 is fixed with respect to the mouthpiece 409 (i.e. so as to move with the mouthpiece 409 between the activated and deactivated states discussed above).

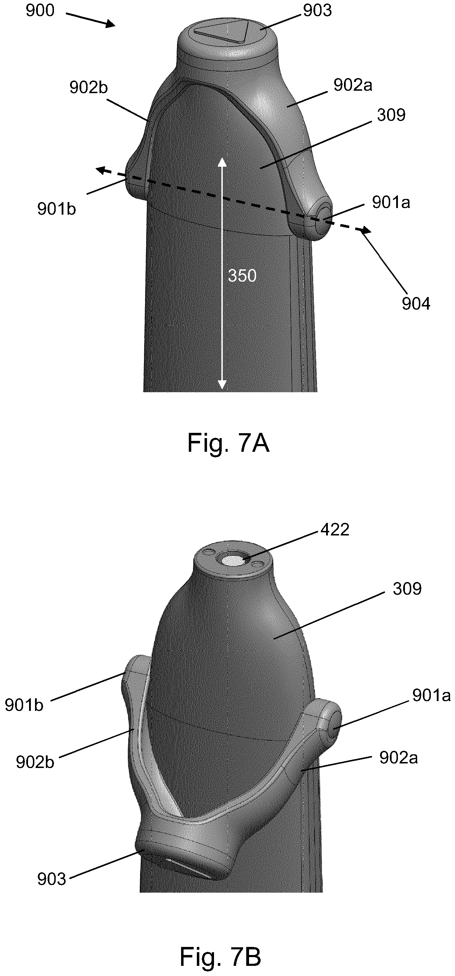

Figure 7A shows a perspective view of a consumable according to the present disclosure. The consumable includes a hinged cap 900 located on an exterior of the consumable. The hinged cap includes protrusions 901a and 901b, which extend from an outer housing of the consumable. Around each protrusion is an arm 902a / 902b, which is rotatable relative to the protrusion and which extends away from the protrusion towards an upper end of the consumable. Each arm widens as it projects from the protrusion, and at the end of both arms is a cap portion 903. The cap portion 903 wider than the mouthpiece 309/409, but only slightly, such that a snug interference fit is achieved between the cap portion and the mouthpiece.

The hinged cap is rotatable around axis 904, which is transversal to the central longitudinal axis 350 discuss above. Accordingly, the cap is rotatable from the sealed configuration shown in Figure 7A in which the mouthpiece 309/409 is sealed from the outside of the aerosol delivery device to an open configuration shown in Figure 7B. In this configuration, the aerosol generating portion 322/422 is exposed to the outside environment, and the user can operate the aerosol delivery device. The hinged cap, and notably the arms 902a/902b and cap portion 903 are formed from a resiliently deformable material such as silicone. This allows the cap to fit snugly over the mouthpiece whilst also allowing it to easily be removed through deformation.

The hinged cap 900 of Figures 7A and 7B is usable in combination with any of the consumables disclosed herein. Various changes to the described embodiments may be made without departing from the scope of the invention as defined in the claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device comprising:
a mouthpiece (309) comprising an end surface at a longitudinal end of the mouthpiece, and a mouthpiece aperture (307) formed in the end surface;
an aerosolisation chamber (325) extending longitudinally into the device from the mouthpiece aperture (307);
a storage chamber for storing an aerosol precursor; and
a porous liquid transfer element (315) comprising an aerosol generating portion (322) at least partly received in the aerosolisation chamber (319) and a conveying portion (317) for conveying liquid from the storage chamber (316) to the aerosol generating portion (322),
wherein the aerosol generating portion of the liquid transfer element is a passive aerosol generating portion,
**characterized in that**
a transverse cross-sectional area of the aerosolisation chamber (319) is uniform along a longitudinal length of the aerosolisation chamber (319),
and
the aerosol generating portion (322) has a larger transverse cross-sectional area than a transverse cross-sectional area of the conveying portion (317) to define a constricted airflow passage between a wall defining the aerosolisation chamber (319) and the liquid transfer element (315).

2. An aerosol delivery device according to claim 1 wherein a transversely extending transition surface (336) is defined between the conveying portion (317) and the aerosol generating portion (322).

3. An aerosol delivery device according to claim 2 wherein the transition surface (336) comprises a concave profile.

4. An aerosol delivery device according to any one of the preceding claims wherein the aerosolisation chamber (319) has a cylindrical shape.

5. An aerosol delivery device according to any one of the preceding claims where a distal transversely extending end surface (329) of the aerosol generating portion (322) is substantially planar.

6. An aerosol delivery device according to claim 5 wherein the planar transversely extending end surface (329) is spaced from the mouthpiece aperture (307) in an upstream longitudinal direction.

7. An aerosol delivery device according to claim 6 wherein the spacing of the transversely extending end surface (329) from the mouthpiece aperture (307) is less than 1 mm.

8. An aerosol delivery device according to any one of the preceding claims wherein the aerosol generating portion (322) is fully received in the aerosolisation chamber (319).

9. An aerosol delivery device according to claim 8 wherein an airflow path through the aerosolisation chamber (319) is defined between the aerosol generating portion (322) and one or more walls defining the aerosolisation chamber (319), the airflow path comprising a constricted region defining the narrowest part of the airflow path.

10. An aerosol delivery device according to claim 9 wherein the airflow path comprises an expansion region downstream of the constricted region.

11. An aerosol delivery device according to claim 10 wherein the cross-sectional area of the airflow path in the expansion region increase in the downstream direction.

12. An aerosol delivery device according to any one of the preceding claims comprising an inlet and a flow passage fluidly connecting the inlet to the aerosolisation chamber (319).

13. An aerosol delivery device according to any one of the preceding claims further comprising a cartomizer (301) comprising a vaporiser in fluid communication with the inlet of the flow passage for delivering a vapour to the flow passage.

14. An aerosol delivery device system comprising an aerosol delivery device according to any one of the preceding claims and a base unit comprising a power source.

## Patentansprüche

1. Aerosolabgabevorrichtung, umfassend:
ein Mundstück (309), das eine Endfläche an einem länglichen Ende des Mundstücks umfasst, und eine in der Endfläche ausgebildete Mundstücköffnung (307);
eine Aerosolisierungskammer (325), die sich von der Mundstücköffnung (307) aus der Länge nach in die Vorrichtung hinein erstreckt;
eine Speicherkammer zum Speichern eines Aerosolvorläufers; und
ein poröses Flüssigkeitstransportelement (315), das einen Aerosolerzeugungsabschnitt (322), welcher zumindest teilweise in der Aerosolisierungskammer (319) aufgenommen ist, und einen Förderabschnitt (317) zum Befördern von Flüssigkeit aus der Speicherkammer (316) zum Aerosolerzeugungsabschnitt (322) umfasst,
wobei der Aerosolerzeugungsabschnitt des Flüssigkeitstransportelements ein passiver Aerosolerzeugungsabschnitt ist,
**dadurch gekennzeichnet, dass** die Querschnittsfläche der Aerosolisierungskammer (319) entlang der longitudinalen Länge der Aerosolisierungskammer (319) gleichbleibend ist,
dass der Aerosolerzeugungsabschnitt (322) eine größere Querschnittsfläche als eine Querschnittsfläche des Förderabschnitts (317) aufweist, um einen eingeengten Luftstromdurchgang zwischen einer die Aerosolisierungskammer (319) definierenden Wand und dem Flüssigkeitstransportelement (315) zu definieren.

2. Aerosolzufuhrvorrichtung nach Anspruch 1, wobei eine sich quer erstreckende Übergangsfläche (336) zwischen dem Förderabschnitt (317) und dem Aerosolerzeugungsabschnitt (322) definiert ist.

3. Aerosolzufuhrvorrichtung nach Anspruch 2, wobei die Übergangsfläche (336) ein konkaves Profil umfasst.

4. Aerosolzufuhrvorrichtung nach einem der vorangegangenen Ansprüche, wobei die Aerosolisierungskammer (319) eine zylindrische Form aufweist.

5. Aerosolzufuhrvorrichtung nach einem der vorangegangenen Ansprüche, wobei eine sich quer erstreckende, distale Endfläche (329) des Aerosolerzeugungsabschnitts (322) im Wesentlichen planar ist.

6. Aerosolzufuhrvorrichtung nach Anspruch 5, wobei die sich quer erstreckende, planare Endfläche (329) von der Mundstücköffnung (307) in eine Stromauf-Längsrichtung beabstandet ist.

7. Aerosolzufuhrvorrichtung nach Anspruch 6, wobei die Beabstandung der sich quer erstreckenden Endfläche (329) von der Mundstücköffnung (307) weniger als 1 mm beträgt.

8. Aerosolzufuhrvorrichtung nach einem der vorangegangenen Ansprüche, wobei der Aerosolerzeugungsabschnitt (322) vollständig in der Aerosolisierungskammer (319) aufgenommen ist.

9. Aerosolzufuhrvorrichtung nach Anspruch 8, wobei zwischen dem Aerosolerzeugungsabschnitt (322) und einer oder mehreren die Aerosolisierungskammer (319) definierenden Wand/Wände ein Luftstrompfad durch die Aerosolisierungskammer (319) definiert ist, wobei der Luftstrompfad einen eingeengten Bereich, der den schmalsten Teil des Luftstrompfads definiert, umfasst.

10. Aerosolzufuhrvorrichtung nach Anspruch 9, wobei der Luftstrompfad einen Expansionsbereich stromabwärts des eingeengten Bereichs umfasst.

11. Aerosolzufuhrvorrichtung nach Anspruch 10, wobei die Querschnittsfläche des Luftstrompfads im Expansionsbereich in die Stromabwärtsrichtung größer wird.

12. Aerosolzufuhrvorrichtung nach einem der vorangegangenen Ansprüche, umfassend einen Einlass und einen Strömungsdurchgang, welcher den Einlass fluidisch mit der Aerosolisierungskammer (319) verbindet.

13. Aerosolzufuhrvorrichtung nach einem der vorangegangenen Ansprüche, ferner umfassend einen Cartomizer (301), der einen Verdampfer umfasst, welcher in Fluidkommunikation mit dem Einlass der Strömungsdurchgangs steht, um dem Strömungsdurchgang Dampf zuzuführen.

14. Aerosolzufuhrvorrichtungssystem, umfassend eine Aerosolzufuhrvorrichtung nach einem der vorangegangenen Ansprüche und eine Basiseinheit, welche eine Leistungsquelle umfasst.

## Revendications

1. Dispositif de distribution d'aérosol, comprenant :
un embout buccal (309) comprenant une surface d'extrémité au niveau d'une extrémité longitudinale de l'embout buccal, et une ouverture d'embout buccal (307) formée dans la surface d'extrémité ;
une chambre d'aérosolisation (325) s'étendant longitudinalement dans le dispositif à partir de l'ouverture d'embout buccal (307) ;
une chambre de stockage pour stocker un précurseur d'aérosol ; et
un élément de transfert de liquide poreux (315) comprenant une partie de génération d'aérosol (322) au moins partiellement reçue dans la chambre d'aérosolisation (319) et une partie de transport (317) pour transporter du liquide depuis la chambre de stockage (316) vers la partie de génération d'aérosol (322),
dans lequel la partie de génération d'aérosol de l'élément de transfert de liquide est une partie de génération d'aérosol passive,
**caractérisé en ce qu'**une aire de section transversale de la chambre d'aérosolisation (319) est uniforme le long d'une longueur longitudinale de la chambre d'aérosolisation (319), et
la partie de génération d'aérosol (322) présente une aire de section transversale plus grande qu'une aire de section transversale de la partie de transport (317) pour définir un passage d'écoulement d'air restreint entre une paroi définissant la chambre d'aérosolisation (319) et l'élément de transfert de liquide (315).

2. Dispositif de distribution d'aérosol selon la revendication 1, dans lequel une surface de transition s'étendant transversalement (336) est définie entre la partie de transport (317) et la partie de génération d'aérosol (322).

3. Dispositif de distribution d'aérosol selon la revendication 2, dans lequel la surface de transition (336) comprend un profil concave.

4. Dispositif de distribution d'aérosol selon l'une quelconque des revendications précédentes, dans lequel la chambre d'aérosolisation (319) présente une forme cylindrique.

5. Dispositif de distribution d'aérosol selon l'une quelconque des revendications précédentes, dans lequel une surface d'extrémité distale s'étendant transversalement (329) de la partie de génération d'aérosol (322) est sensiblement plane.

6. Dispositif de distribution d'aérosol selon la revendication 5, dans lequel la surface d'extrémité plane s'étendant transversalement (329) est espacée de l'ouverture d'embout buccal (307) dans une direction longitudinale amont.

7. Dispositif de distribution d'aérosol selon la revendication 6, dans lequel l'espacement de la surface d'extrémité s'étendant transversalement (329) par rapport à l'ouverture d'embout buccal (307) est inférieur à 1 mm.

8. Dispositif de distribution d'aérosol selon l'une quelconque des revendications précédentes, dans lequel la partie de génération d'aérosol (322) est complètement reçue dans la chambre d'aérosolisation (319).

9. Dispositif de distribution d'aérosol selon la revendication 8, dans lequel un trajet d'écoulement d'air à travers la chambre d'aérosolisation (319) est défini entre la partie de génération d'aérosol (322) et une ou plusieurs parois définissant la chambre d'aérosolisation (319), le trajet d'écoulement d'air comprenant une région rétrécie définissant la partie la plus étroite du trajet d'écoulement d'air.

10. Dispositif de distribution d'aérosol selon la revendication 9, dans lequel le trajet d'écoulement d'air comprend une région d'expansion en aval de la région rétrécie.

11. Dispositif de distribution d'aérosol selon la revendication 10, dans lequel l'aire de section transversale du trajet d'écoulement d'air dans la région d'expansion augmente dans la direction aval.

12. Dispositif de distribution d'aérosol selon l'une quelconque des revendications précédentes, comprenant une entrée et un passage d'écoulement reliant l'entrée à la chambre d'aérosolisation (319) de manière fluidique.

13. Dispositif de distribution d'aérosol selon l'une quelconque des revendications précédentes, comprenant en outre un cartomiseur (301) comprenant un vaporisateur en communication fluidique avec l'entrée du passage d'écoulement pour distribuer une vapeur au passage d'écoulement.

14. Système de distribution d'aérosol comprenant un dispositif de distribution d'aérosol selon l'une quelconque des revendications précédentes et une unité de base comprenant une source d'alimentation.
